# EUROPEAN PATENT SPECIFICATION

(11) **EP 0 278 121 B1**
(45) Date of publication and mention of the grant of the patent: **06.09.1995**
(21) Application number: 87202220.7
(22) Date of filing: 16.09.1982
(51) Int. Cl.: C12N 15/00, C12P 21/02

(54) **Microbially produced bovine growth hormone (BGH) and its use**
Mikrobisch-produziertes Rinderwachstumshormon und dessen Verwendung
Hormone de croissance bovine produite biologiquement et son utilisation

(30) Priority: 18.09.1981 US 303687
(43) Date of publication of application: 17.08.1988
(62) Divisional of application: 82304880.6
(73) Proprietor: GENENTECH, INC., South San Francisco California 94080 (US)
(72) Inventor: De Boer, Herman Albert, NL-2371 GD Roelofarendsveen (NL); Seeburg, Peter H., 69 Heidelberg (DE); Heyneker, Herbert L., Hillsborough, California 94010 (US)
(74) Representative: Armitage, Ian Michael

(56) References cited:
- EP-A- 0 047 600
- EP-A- 0 067 026
- EP-A- 0 068 646
- GB-A- 2 073 245
- CHEMICAL ABSTRACTS, vol. 93, no. 19, 10th November 1980, page 208, abstract no. 181237w, Columbus, Ohio, US; W.L. Miller et al.: "Molecular cloning of DNA complementary to bovine growth hormone mRNA"
- NUCLEIC ACIDS RESEARCH, vol. 9, no. 1, 1981, pages 19-30, IRL Press Ltd, London, GB; E. KESHET et al.: "Cloning of bovine growth hormone gene and its expression in bacteria"

## Description

This invention relates to bovine growth hormone. It is divided out of our European application EP75444 which discloses a method of obtaining improved microbial expression of exogenous polypeptides, of which BGH is exemplified as the exogenous polypeptide. The technological background and general procedures to be adopted are therefore described in the parent specification and are not repeated in detail here. Reference may be made to the parent specification as appropriate.

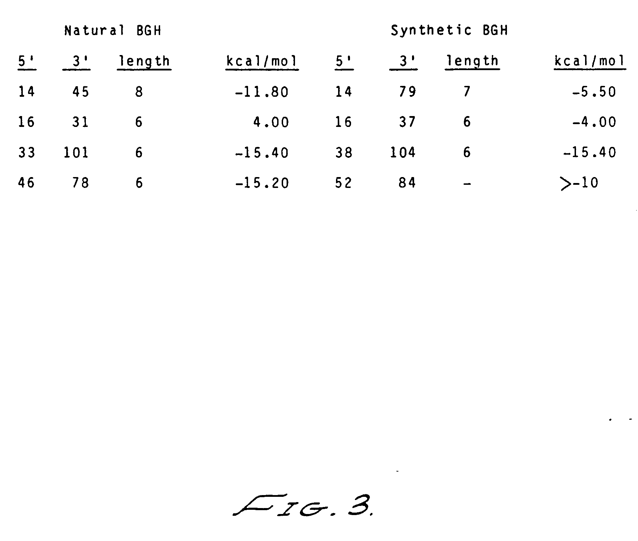

BGH is endogenous in cattle and is responsible for proper physical maturation of the animal. It is also useful for increasing weight gain, feed conversion efficiency, lean to fat ratio, and milk production. Its sequence of 190 amino acids is known. See Dayhoff, Atlas of Protein Sequence and Structure 1972, National Biomedical Research Foundation, Washington, D.C. The present invention makes possible the preparation of commercial quantities of the compound, enabling now its application on a large scale in the animal husbandry industry. An initial approach toward preparing BGH microbially took advantage of a source of bovine pituitary glands. By extraction and purification, the requisite mRNA for BGH was isolated and from it, corresponding cDNA prepared. Thus, this initial work resulted in a gene corresponding, for all intents and purposes, to the natural DNA sequence of BGH. After removal of DNA coding for the presequence and adding a start codon, the cDNA was ligated to a plasmid vector under proper control of a promoter. This plasmid was used to transform E. coli host which has grown under usual conditions. The efficiency of expression of BGH product was poor, a consequence, it was discovered, of conformational structure of the messenger RNA, which greatly reduced its accessibility for ribosomal translation, cf. Figure 3.

For example, it was found that in "natural" BGH mRNA there are regions of complementary homology. One significant region centers around positions +46 to +51 with a homologous region at positions +73 to +78. Secondary structure considerations, in these two defined regions, are thought to create a hairpin arrangement just downstream from the translation start codon ATG and the ribosome binding site. This conformational arrangement interferes with or prematurely disrupts ribosomal binding, and hence, inhibits translation.

The recognition of this phenomenon prompted investigations into the nature of the DNA sequence in these regions and the discovery of methods and means to obviate the problem.

The parent invention is based upon the discovery that the presence of secondary/tertiary conformational structure in the mRNA interferes with the initiation and maintenance of ribosomal binding during the translation phase of heterologous gene expression.

The parent invention is particularly directed to the use of synthetically derived gene insert portions that are prepared so as to both encode the desired polypeptide product and provide mRNA that has minimal secondary/tertiary structure and hence is accessible for efficient ribosomal translation.

According to the procedure of the parent invention, synthetic DNA is provided for a substantial portion of the initial coding sequence of a heterologous gene insert, and optionally, upstream therefrom through the ATG translational start codon and ribosome binding site. The critical portion of DNA is chemically synthesized, keeping in mind two factors: 1) the creation of a sequence that codes for the initial (N-terminal) amino acid sequence of a polypeptide comprising a functional protein or bioactive portion thereof and 2) the assurance that said sequence provides, on transcription, messenger RNA that has a secondary/tertiary conformational structure which is insufficient to interfere with its accessibility for efficient ribosomal translation, as herein defined. Such chemical synthesis may use standard organic synthesis using modified mononucleotides as building blocks such as according to the method of Crea et al., Nucleic Acids Research 8, 2331 (1980) and/or the use of site directed mutagenesis of DNA fragments such as according to the method of Razin et al., Proc. Natl. Acad. Sci. (USA) 75, 4268 (1978) and/or synthetic primers on certain appropriately sequenced DNA fragments followed by specific cleavage of the desired region.

The parent invention is directed to a process of preparing DNA sequences comprising nucleotides arranged sequentially so as to encode the proper amino acid sequence of a given polypeptide. This method may involve obtaining a substantial portion of the DNA coding sequence of a given polypeptide via means other than chemical synthesis, most often by reverse transcription from requisite tissue and/or culture cell messenger RNA. This fragment encodes the C-terminal portion of the polypeptide and is ligated, to a remainder of the coding sequence, e.g. obtained by chemical synthesis, optionally including properly positioned translational start and stop signals and upstream DNA through the ribosome binding site and the first nucleotide (+1) of the resultant messenger RNA. The synthetic fragment is designed by nucleotide choice dependent on conformation of the corresponding messenger RNA according to the criteria as herein discussed.

The thus prepared DNA sequences are suited for insertion and use in replicable expression vehicles designed to direct the production of the heterologous polypeptide in a transformant microorganism. In these vehicles, the DNA sequence is operably linked to promotor systems which control its expression. The invention is further directed to the replicable expression vehicles and the transformant microorganisms so produced as well as to cultures of these microorganisms in fermentation media.

The chemically synthetic DNA sequences extend preferably from the ATG translation initiation site, and optionally upstream therefrom a given distance, to or beyond the transcription initiation site (labelled +1 by convention), and to sequences downstream encoding a substantial part of the desired polypeptide. By way of preference, the synthetic DNA comprises upwards of approximately 75 or more nucleotide pairs of the structural gene representing about the proximal (N-terminal) 25 amino acids of the intended polypeptide. In particularly preferred embodiments, the synthetic DNA sequence extends from about the translation initiation site (ATG) to about nucleotide 75 of the heterologous gene. In alternative terms, the synthetic DNA sequence comprises nucleotide pairs from +1 (transcription initiation) to about nucleotide 100 of the transcript.

Because of the degeneracy of the genetic code, there is substantial freedom in codon choice for any given amino acid sequence. Given this freedom, the number of different DNA nucleotide sequences encoding any given amino acid sequence is exceedingly large, for example, upwards of 2.6 x 10⁵ possibilities for somatostatin consisting of only 14 amino acids. Again, the parent invention provides methods and means for selecting certain of these DNA sequences, those which will efficiently prepare functional product. For a given polypeptide product hereof, the parent invention provides means to select, from among the large number possible, those DNA sequences that provide transcripts, the conformational structure of which admits of accessibility for operable and efficient ribosomal translation.

Conformational structure of mRNA transcripts is a consequence of hydrogen bonding between complementary nucleotide sequences that may be separated one from another by a sequence of noncomplementary nucleotides. Such bonding is commonly referred to as secondary structure. So-called tertiary structures may add to the conformation of the overall molecule. These structures are believed to be a result of spatial interactions within one or more portions of the molecule - so-called stacking interactions. In any event, the conformational structure of a given mRNA molecule can be determined and measured. Furthermore, we have discovered that certain levels of conformational structure of mRNA transcripts interfere with efficient protein synthesis, thus effectively blocking the initiation and/or continuation of translation (elongation) into polypeptide product. Accordingly, levels at which such conformational structure does not occur, or at least is minimal, can be predicted. Nucleotide choice can be prescribed on the basis of the predictable, permissible levels of conformational structure, and preferred gene sequences determined accordingly.

The measurement of mRNA conformational structure is determined, in accordance herewith, by measuring the energy levels associated with the conformational structure of the mRNA molecule.

In determining such energy levels, the thermodynamic disassociation energy connected with one or a series of homologous base pairings is calculated, for example according to the rules of Tinoco et al., Nature New Biol. 246, 40 (1973). In this calculation, AT base pairing is assigned an associated energy level of about -1.2 kcal/mole while a CG base pairing is assigned an associated energy level of about -2 kcal/mole. Adjacent homologous pairings are more than additive, doubtless due to stacking interactions and other associative factors. In any event, it has been determined that in those instances where regional base pairing interactions result in energy levels stronger than about -12 kcal/mole (that is, values expressed arithmetically in numbers less than about -12 kcal/mole) for a given homologous sequence, such interactions are likely sufficient to hinder or block the translation phase of expression, most probably by interfering with accessibility for necessary ribosomal binding.

A given DNA sequence is screened as follows: A first series of base pairs, e.g., approximately the first six base pairs, are compared for homology with the corresponding reverse last base pairs of the gene. If such homology is found, the associate energy levels are calculated according to the above considerations. The first series of base pairs is next compared with the corresponding last base pairs up to the penultimate base pair of the gene and the associative energy levels of any homology calculated. In succession the first series of base pairs is next compared with the corresponding number of base pairs up to the antepenultimate base pair, and so on until the entire gene sequence is compared, back to front. Next, the series of base pairs beginning one downstream from the first series, e.g. base pairs 2 to 7 of the prior example, is compared with the corresponding number from the end and progressively toward the front of the gene, as described above. This procedure is repeated until each base pair is compared for homology with all other regions of the gene and associated energy levels are determined. Thus, for example in Figure 3 there are provided results of such scanning and calculating for two genes - those encoding natural bovine growth hormone (BGH) and synthetic (i.e., hybrid) BGH. It can be seen that natural BGH contains two regions of homology considered relevant herein (i.e., energy level greater than about -12 kcal/mole), to wit, six base pairs from base pair 33 to 38 with homologous pairs 96 to 101 and six base pairs from 46 to 51 with 73 to 78. The first is not significant for present purpose, despite the energy level (-15.40 kcal/mole), presumably because the region of homology lies downstream a sufficient distance so as not to be influential on translation efficiency. The second region is significant as evidenced by the poor yields of product as described herein cf. infra. The synthetic BGH gene where such region of homology was eliminated provided yields of synthetic BGH (N-terminal methionyl BGH) in amounts exceeding 100,000 copies per cell. By virtue of its synthesis in microbial culture this BGH is essentially free of other proteins of bovine origin.

The present invention therefore provides, in various aspects, BGH essentially free of other proteins of bovine origin, a microorganism capable of producing BGH in amounts exceeding 100 thousand copies per cell, cell-free N-terminal methionyl BGH, a composition comprising a microorganism and N-terminal methionyl BGH, N-methionyl forms of BGH, and industrially applicable uses of the latter, especially in increasing milk production in cattle.

An embodiment of the present invention will now be described by way of example with reference to the accompanying drawings, in which:

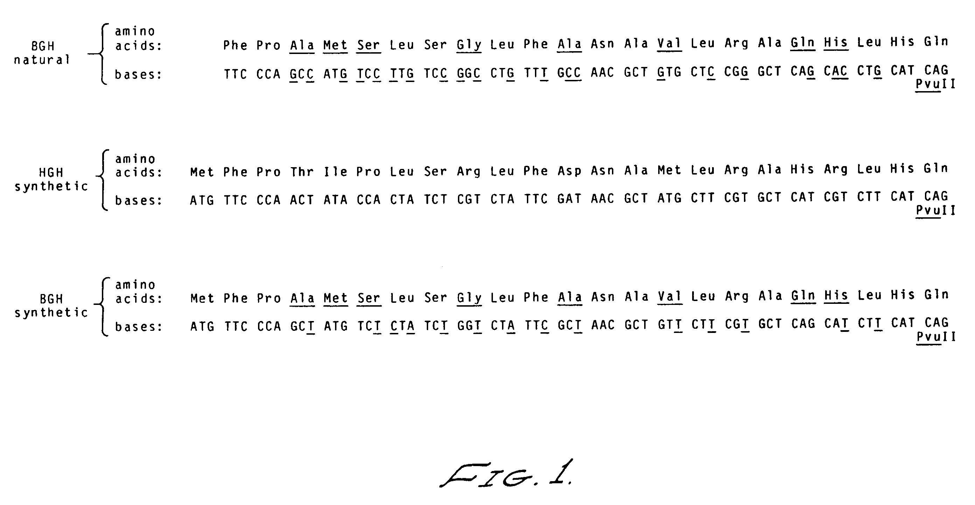

Figure 1 depicts the amino acid and nucleotide sequences of the proximal portions of a natural BGH, synthetic HGH, and synthetic BGH. The amino acids and nucleotides in natural BGH that are different from those in synthetic HGH are underlined. The nucleotides in the proximal portion of the synthetic BGH gene that differ from those in the natural BGH gene also are underlined. The position of the Pvu II restriction site at the end of the proximal portion of these genes is indicated.
   In arriving at the synthetic BGH gene encoding the proper amino acid sequence for BGH, the nucleotide sequences of natural BGH and synthetic HGH were compared. Nucleotide selections were made based upon the synthetic HGH gene for construction of the synthetic BGH gene taking into account also the latitude permitted by the degeneracy of the genetic code, using a minimum of nucleotide changes from the synthetic HGH sequence.

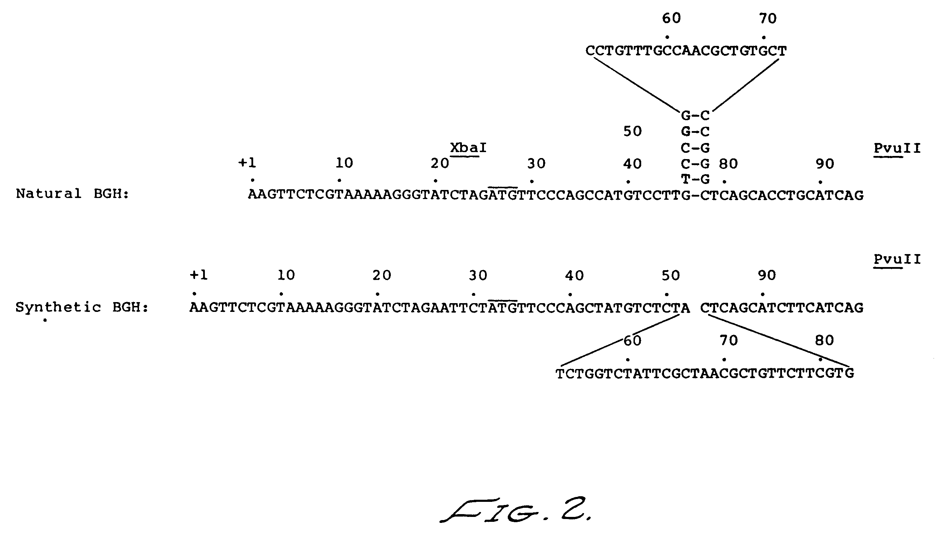

Figure 2 depicts the nucleotide sequences of the sense strands of both natural and synthetic BGH genes along with the transcribed portions of the respective preceding trp-promotor sequences. The first nucleotide of each transcript is indicated as "+1" and the following nucleotides are numbered sequentially. The sequences are lined up to match the translated coding regions of both genes, beginning at the start codon "ATG" of each (overlined). The transcript of the natural BGH gene shows an area of "secondary structure" due to interactions of nucleotides 46 to 51 with nucleotides 73 to 78, respectively (see Figure 3), thus creating the stem-loop structure depicted. This area is not present in the synthetic BGH gene, removed by virtue of nucleotide changes (see Figure 1), which nevertheless retains the correct amino acid sequence.
Figure 3 shows the locations and stabilities of secondary structures in the transcripts of natural and synthetic BGH. (See Figure 2). These locations and stabilities were determined using a nucleotide by nucleotide analysis, as described herein. Each area of significant secondary structure of each proximal portion of gene is listed in the respective table. Thus, for natural BGH versus synthetic BGH, it is noted that the energy levels of "secondary structure" at corresponding portions of the translatable transcripts (namely, nucleotides 46 to 78 comprising a 6 nucleotide long stem in natural BGH versus nucleotides 52 to 84 of synthetic BGH) are markedly different (-15.2 kcal/mole versus greater than -10 kcal/mole), accounting for the observed success of expression of the synthetic BGH gene versus the natural BGH gene, cf. infra. The energy levels indicate the significance of the relative amounts of tolerable "secondary structure", i.e., values arithmetically greater than about -12 kcal/mole based upon thermodynamic energy considerations. The significance of location of "secondary structure" can be appreciated by the fact that energy levels calculated for positions 33 to 101 versus 38 to 104 of natural versus synthetic BGH, respectively, did not significantly influence expression levels.
Figure 4 depicts the construction of pBGH 33 used as shown in Figure 5.
Figure 5 depicts the construction of plasmids harboring DNA sequences for hybrid polypeptides: pBHGH 33-1 used as shown in Figure 7, pBHGH being a hybrid of bovine and human growth hormone sequences, and pHBGH a hybrid of human and bovine sequences.
Figure 6 depicts the technique used to assemble the synthetic proximal portion of the BGH gene, pBR 322-01, used in the construction shown in Figure 7.
Figure 7 depicts the construction of the plasmid (pBGH 33-3) harboring the gene for BGH comprising the synthetic proximal portion as shown in Figure 6.
Figure 8 depicts the construction of expression plasmid pBGH 33-4 harboring the hybrid BGH gene.
Figure 9 is the result of a polyacrylamide gel segregation of cell protein. Part A shows no BGH production at any cell density using the culture containing natural BGH gene. Part B shows the expression of synthetic BGH gene (lanes BGH #1 and #2) in the same medium as used for Part A. The levels of expression indicated in Part B, as opposed to Part A, reflect the production of BGH in amounts exceeding about 100 thousand copies per cell.

Advantage was taken of a Pvu II endonuclease restriction site at the BGH DNA corresponding to amino acid 24. DNA for the first 24 amino acids of BGH was chemically synthesized, the selection of nucleotides taking into strict account proper coding sequence and resultant mRNA secondary/tertiary structure considerations. Employing the method defined above, it was found that certain nucleotide base selections would be suitable, on the basis of predicted conformational structure energy levels, to prepare gene sequences properly encoding BGH but devoid of problematic conformational structure. One of these was selected and synthesized. Ligations at the Pvu II terminus of the synthetic piece to the cDNA downstream therefrom produced the desired hybrid gene. Construction of a replicable expression vector containing said heterologous gene as an operable insert successfully resulted in efficient expression of BGH in transformed E. coli host.

### Detailed Description

### Synthesis of Proximal Portion of BGH Gene

Twelve fragments, U 1-6 (upper strand) and L 1-6 (lower strand), were synthesized. Also synthesized, in order to repair the 3' end of the gene, were 2 fragments, BGH Repair (1) (upper strand) and BGH Repair (2) (lower strand).

The 14 fragments were synthesized according to the method of Crea et al., Nucleic Acids Research, 8, 2331 (1980). The syntheses of the fragments were accomplished from the appropriate solid support (cellulose) by sequential addition of the appropriate fully protected dimer - or trimer- blocks. The cycles were carried out under the same conditions as described in the synthesis of oligothymidilic acid (see Crea et al., Supra.) The final polymer was treated with base (aq. conc NH₃) and acid (80%v/v aq. HOAC), the polymer pelleted off and the supernatant evaporated to dryness. The residue, as dissolved in 4% v/v aq. NH₃, was washed with ether (3x) and used for the isolation of the fully deprotected fragment. Purification was accomplished by hplc on Rsil NH₂ u-particulate column. Gel electrophoretic analysis showed that each of the fragments, U,L 1-6 and BGH Repair (1) and (2), had the correct size:

The complete nucleotide (and deduced amino acid) sequence of the thus constructed hybrid BGH gene is as follows:

1
met phe pro ala met ser leu ser gly leu phe ala asn ala val
ATG TTC CCA GCT ATG TCT CTA TCT GGT CTA TTC GCT AAC GCT GTT

20
leu arg ala gln his leu his gln leu ala ala asp thr phe lys
CTT CGT GCT CAG CAT CTT CAT CAG CTG GCT GCT GAC ACC TTC AAA

40
glu phe glu arg thr tyr ile pro glu gly gln arg tyr ser ile
GAG TTT GAG CGC ACC TAC ATC CCG GAG GGA CAG AGA TAC TCC ATC

gln asn thr gln val ala phe cys phe ser glu thr ile pro ala
CAG AAC ACC CAG GTT GCC TTC TGC TTC TCT GAA ACC ATC CCG GCC

60
pro thr gly lys asp glu ala gln gln lys ser asp leu glu leu
CCC ACG GGC AAG GAT GAG GCC CAG CAG AAA TCA GAC TTG GAG CTG

80
leu arg ile ser leu leu leu ile gln ser trp leu gly pro leu
CTT CGC ATC TCA CTG CTC CTC ATC CAG TCG TGG CTT GGG CCC CTG

100
gln phe leu ser arg val phe thr asn ser leu val phe gly thr
CAG TTC CTC AGC AGA GTC TTC ACC AAC AGC TTG GTG TTT GGC ACC

ser asp arg val tyr glu lys leu lys asp leu glu glu gly ile
TCG GAC CGT GTC TAT GAG AAG CTG AAG GAC CTG GAG GAA GGC ATC

120
leu ala leu met arg glu leu glu asp gly thr pro arg ala gly
CTG GCC CTG ATG CGG GAG CTG GAA GAT GGC ACC CCC CGG GCT GGG

140
gln ile leu lys gln thr tyr asp lys phe asp thr asn met arg
CAG ATC CTC AAG CAG ACC TAT GAC AAA TTT GAC ACA AAC ATG CGC

160
ser asp asp ala leu leu lys asn tyr gly leu leu ser cys phe
AGT GAC GAC GCG CTG CTC AAG AAC TAC GGT CTG CTC TCC TGC TTC

arg lys asp leu his lys thr glu thr tyr leu arg val met lys
CGG AAG GAC CTG CAT AAG ACG GAG ACG TAC CTG AGG GTC ATG AAG

180 190
cys arg arg phe gly glu ala ser cys ala phe stop
TGC CGC CGC TTC GGG GAG GCC AGC TGC GCA TTC TAG

| Fragment | Sequence | Size |
|---|---|---|
| U 1 | ^{5'} AAT.TCT.ATG.TTC.C^{3'} | 13-mer |
| U 2 | ^{5'} CAG.CTA.TGT.CTC.T^{3'} | 13-mer |
| U 3 | ^{5'} ATC.TGG.TCT.ATT.C^{3'} | 13-mer |
| U 4 | ^{5'} GCT.AAC.GCT.GTT.C^{3'} | 13-mer |
| U 5 | ^{5'} TTC.GTG.CTC.AGC.A^{3'} | 13-mer |
| U 6 | ^{5'} TCT.TCA.TCA.GCT.GA^{3'} | 14-mer |
| L 1 | ^{5'} ATA.GCT.GGG.AAC.ATA.G^{3'} | 16-mer |
| L 2 | ^{5'} ACC.AGA.TAG.AGA.C^{3'} | 13-mer |
| L 3 | ^{5'} CGT.TAG.CGA.ATA.G^{3'} | 13-mer |
| L 4 | ^{5'} GCA.CGA.AGA.ACA.G^{3'} | 13-mer |
| L 5 | ^{5'} ATG.AAG.ATG.CTG.A^{3'} | 13-mer |
| L 6 | ^{5'} AGC.TTC.AGC.TG^{3'} | 11-mer |
| BGH Repair (1) | ^{5'} AA.TTC.AGC.TGC.GCA.TTC.TAG.A^{3'} | 21-mer |
| BGH Repair (2) | ^{5'} AG.CTT.CTA.GAA.TGC.GCA.GCT.G^{3'} | 21-mer |

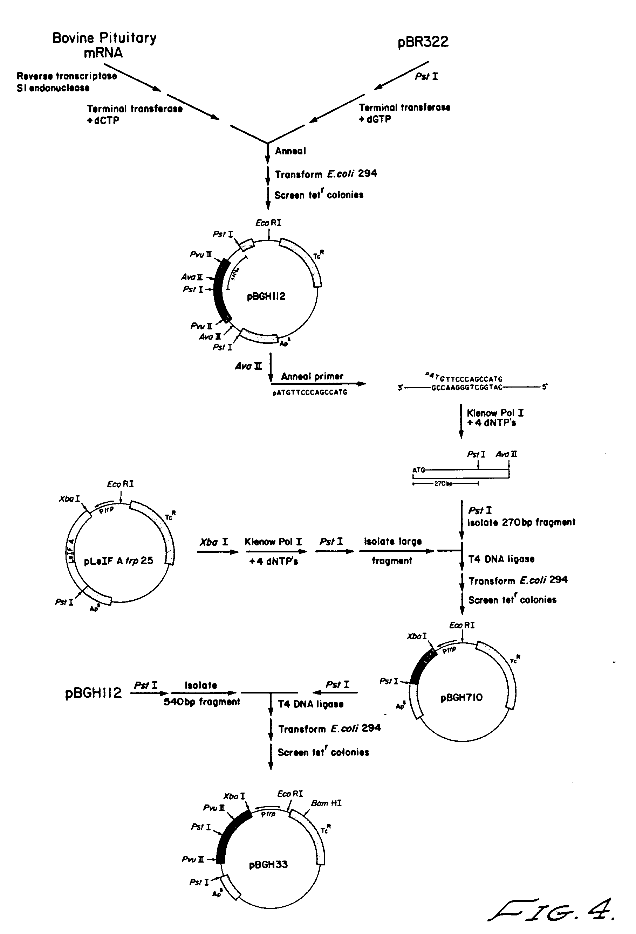

### Construction of pBGH 33 (Fig. 4)

Fresh frozen bovine pituitaries were macerated and RNA was extracted by the guanidium thiocyanate method. (Harding et al., J. Biol Chem. 252 (20), 7391 (1977) and Ullrich et al., Science 196, 1313 (1977)). The total RNA extract was then passed over an oligo-dT cellulose column to purify poly A containing messenger RNA (mRNA). Using reverse transcriptase and oligo-dT as a primer, single stranded cDNA was made from the mRNA. Second strand synthesis was achieved by use of the Klenow fragment of DNA polymerase I. Following S1 enzyme treatment and acrylamide gel electrophoresis a size cut of the total cDNA (ca. 500-1500 bp) was eluted and cloned into the Pst I site of the amp^{R} gene of pBR 322 using traditional tailing and annealing conditions.

The pBR 322 plasmids containing cDNA were transformed into E. coli K-12 strain 294 (ATCC No. 31446). Colonies containing recombinant plasmids were selected by their resistance to tetracycline and sensitivity to ampicillin. Approximately 2000 of these clones were screened for BGH by colony hybridization.

The cDNA clones of HGH contain an internal 550 bp HaeIII fragment. The amino acid sequence of this region is very similar to the BGH amino acid sequence. This HGH HaeIII fragment was radioactively labeled and used as a probe to find the corresponding BGH sequence amongst the 2000 clones.

Eight positive clones were identified. One of these, pBGH112, was verified by sequence analysis as BGH. This full-length clone is 940 bp long containing the coding region of the 26 amino acid presequence as well as the 191 amino acid protein sequence.

In order to achieve direct BGH expression, a synthetic "expression primer" was made having the sequence 5ʹ-ATGTTCCCAGCCATG-3ʹ. The nucleotides in the fourth through fifteenth position are identical to the codons of the first 4 amino acids of the mature BGH protein, as determined by sequence data of pBGH 112 . Only the 5ʹ ATG (methionine) is alien to this region of the protein. This was necessary in order to eliminate the presequence region of our BGH clone and to provide the proper initiation codon for protein synthesis. By a series of enzymatic reactions this synthetic primer was elongated on the BGH 112 cDNA insert. The primed product was cleaved with Pst I to give a DNA fragment of 270 bp containing coding information up to amino acid 90. (Figure 4) This "expression" BGH cDNA fragment was ligated into a pBR 322 vector which contained the trp promotor. This vector was derived from pLeIF A trp25 (Goeddel et al., Nature 287, 411 (1980)). The interferon cDNA was removed and the trp25-322 vector purified by gel electrophoresis. The recombinant plasmid (pBGH710) now contained the coding information for amino acids 1-90 of the mature BGH protein, linked directly to the trp promotor. This linkage was verified by DNA sequence analysis. The second half of the coding region and the 3ʹ untranslated region was isolated from pBGH112 by PstI restriction digest and acrylamide gel electrophoresis. This "back-end" fragment of 540 bp was then ligated into pBGH710 at the site of amino acid 90. Recombinant plasmids were checked by restriction analysis and DNA sequencing. The recombinant plasmid, pBGH33, has the trp promotor directly linked via ATG with the complete DNA coding sequence for mature BGH.

### Construction of pHGH 207-1

Plasmid pGM1 carried the E. coli tryptophan operon containing the deletion LE1413 (G.F. Miozzari, et al., (1978) J. Bacteriology 1457-1466)) and hence expresses a fusion protein comprising the first 6 amino acids of the trp leader and approximately the last third of the trp E polypeptide (hereinafter referred to in conjunction as LEʹ), as well as the trp D polypeptide in its entirety, all under the control of the trp promoter-operator system. The plasmid, 20 »g, was digested with the restriction enzyme PvuII which cleaves the plasmid at five sites. The gene fragments were next combined with EcoRI linkers (consisting of a self complementary oligonucleotide of the sequence: pCATGAATTCATG) providing an EcoRI cleavage site for a later cloning into a plasmid containing an EcoRI site. The 20 »g of DNA fragments obtained from pGM1 were treated with 10 units T₄ DNA ligase in the presence of 200 pico moles of the 5ʹ-phosphorylated synthetic oligonucleotide pCATGAATTCATG and in 20»l T₄ DNA ligase buffer (20mM tris, pH 7.6, 0.5 mM ATP, 10 mM MgCl₂, 5 mM dithiothreitol) at 4°C overnight. The solution was then heated 10 minutes at 70°C to inactivate ligase. The linkers were cleaved by EcoRI digestion and the fragments, now with EcoRI ends, were separated using polyacrylamide gel electrophoresis (hereinafter "PAGE") and the three largest fragments isolated from the gel by first staining with ethidium bromide, locating the fragments with ultraviolet light, and cutting from the gel the portions of interest. Each gel fragment, with 300 microliters 0.1xTBE, was placed in a dialysis bag and subjected to electrophoresis at 100 V for one hour in 0.1xTBE buffer (TBE buffer contains: 10.8 gm tris base, 5.5 gm boric acid, 0.09 gm Na₂EDTA in 1 liter H₂O). The aqueous solution was collected from the dialysis bag, phenol extracted, chloroform extracted and made 0.2 M sodium chloride, and the DNA recovered in water after ethanol precipitation. (All DNA fragment isolations hereinafter described are performed using PAGE followed by the electroelution method just discussed.) The trp promoter-operator-containing gene with EcoRI sticky ends was identified in the procedure next described, which entails the insertion of fragments into a tetracycline sensitive plasmid which, upon promoter-operator insertion, becomes tetracycline resistant.

Plasmid pBRHl, (R.I. Rodriguez, et al., Nucleic Acids Research 6, 3267-3287 [1979]) expresses ampicillin resistance and contains the gene for tetracycline resistance but, there being no associated promoter, does not express that resistance. The plasmid is accordingly tetracycline sensitive. By introducing a promoter-operator system in the EcoRI site, the plasmid can be made tetracycline resistant.

pBRHl was digested with EcoRI and the enzyme removed by phenol/CHCl₃ extraction followed by chloroform extraction and recovered in water after ethanol precipitation. The resulting DNA molecule was, in separate reaction mixtures, combined with each of the three DNA fragments obtained as described above and ligated with T₄ DNA ligase as previously described. The DNA present in the reaction mixture was used to transform competent E. coli K-12 strain 294 (K. Backman et al., Proc Nat'l Acad Sci USA 73, 4174-4198 (1976) (ATCC no. 31446) by standard techniques (V. Hershfield et al., Proc Nat'l Acad Sci USA 71, 3455-3459 (1974) and the bacteria plated on LB plates containing 20 »g/ml ampicillin and 5 »g/ml tetracycline. Several tetracycline-resistant colonies were selected, plasmid DNA isolated and the presence of the desired fragment confirmed by restriction enzyme analysis. The resulting plasmid, designated pBRHtrp, expresses β-lactamase, imparting ampicillin resistance, and it contains a DNA fragment including the trp promoter-operator and encoding a first protein comprising a fusion of the first six amino acids of the trp leader and approximately the last third of the trp E polypeptide (this polypeptide is designated LEʹ), and a second protein corresponding to approximately the first half of the trp D polypeptide (this polypeptide is designated Dʹ), and a third protein coded for by the tetracycline resistance gene.

pBRH trp was digested with EcoRI restriction enzyme and the resulting fragment 1 isolated by PAGE and electroelution. EcoRI-digested plasmid pSom 11 (K. Itakura et al, Science 198, 1056 (1977); G.B. patent publication no. 2 007 676 A) was combined with this fragment 1. The mixture was ligated with T₄ DNA ligase as previously described and the resulting DNA transformed into E. coli K-12 strain 294 as previously described. Transformant bacteria were selected on ampicillin-containing plates. Resulting ampicillin-resistant colonies were screened by colony hybridization (M. Gruenstein et al., Proc Nat'l Acad Sci USA 72, 3951-3965 [1975]) using as a probe the trp promoter- operator-containing fragment 1 isolated from pBRHtrp, which had been radioactively labelled with P³². Several colonies shown positive by colony hybridization were selected, plasmid DNA was isolated and the orientation of the inserted fragments determined by restriction analysis employing restriction enzymes BglII and BamHI in double digestion. E. coli 294 containing the plasmid designated pSOM7Δ2, which has the trp promoter-operator fragment in the desired orientation was grown in LB medium containing 10 »g/ml ampicillin. The cells were grown to optical density 1 (at 550 nm), collected by centrifugation and resuspended in M9 media in tenfold dilution. Cells were grown for 2-3 hours, again to optical density 1, then lysed and total cellular protein analyzed by SDS (sodium dodecyl sulfate) urea (15 % w/v) polyacrylamide gel electrophoresis (J.V. Maizel Jr. et al., Meth Viral 5, 180-246 (1971)).

The plasmid pSom7Δ2, 10»g, was cleaved with EcoRI and the DNA fragment 1 containing the tryptophan genetic elements was isolated by PAGE and electroelution. This fragment, 2»g, was digested with the restriction endonuclease Taq I, 2 units, 10 minutes at 37°C such that, on the average, only one of the approximately five Taq I sites in each molecule is cleaved. This partially digested mixture of fragments was separated by PAGE and an approximately 300 base pair fragment 2 that contained one EcoRI end and one Taq I end was isolated by electroelution. The corresponding Taq I site is located between the transcription start and translation start sites and is 5 nucleotides upstream from the ATG codon of the trp leader peptide. The DNA sequence about this site is shown in Figure 4. By proceeding as described, a fragment could be isolated containing all control elements of the trp operon, i.e., promoter-operator system, transcription initiation signal, and part of the trp leader ribosome binding site.

The Taq I residue at the 3ʹ end of the resulting fragment adjacent the translation start signal for the trp leader sequence was next converted into an XbaI site. This was done by ligating the Fragment 2 obtained above to a plasmid containing a unique (i.e., only one) EcoRI site and a unique XbaI site. For this purpose, one may employ essentially any plasmid containing, in order, a replicon, a selectable marker such as antibiotic resistance, and EcoRI, XbaI and BamHI sites. Thus, for example, an XbaI site can be introduced between the EcoRI and BamHI sites of pBR322 (F. Bolivar et al., Gene 2, 95-119 [1977]) by, e.g., cleaving at the plasmid's unique Hind III site with Hind III followed by single strand-specific nuclease digestion of the resulting sticky ends, and blunt end ligation of a self annealing double-stranded synthetic nucleotide containing the recognition site such as CCTCTAGAGG. Alternatively, naturally derived DNA fragments may be employed, as was done in the present case, that contain a single XbaI site between EcoRI and BamHI cleavage residues. Thus, an EcoRI and BamHI digestion product of the viral genome of hepatitis B was obtained by conventional means and cloned into the EcoRI and BamHI sites of plasmid pGH6 (D.V. Goeddel et al., Nature 281, 544 [1979])) to form the plasmid pHS32. Plasmid pHS32 was cleaved with XbaI, phenol extracted, chloroform extracted and ethanol precipitated. It was then treated with 1 »l E. coli polymerase I, Klenow fragment (Boehringer-Mannheim) in 30 »l polymerase buffer (50 mM potassium phosphate pH 7.4, 7mM MgCl₂, 1 mM β-mercaptoethanol) containing 0.1mM dTTP and 0.1mM dCTP for 30 minutes at 0°C then 2 hr. at 37°C. This treatment causes 2 of the 4 nuceotides complementary to the 5ʹ protruding end of the XbaI cleavage site to be filled in: 
5' CTAGA—— 5' CTAGA——
3' T—— 3' TCT——
Two nucleotides, dC and dT, were incorporated giving an end with two 5ʹ protruding nucleotides. This linear residue of plasmid pHS32 (after phenol and chloroform extraction and recovery in water after ethanol precipitation) was cleaved with EcoRI. The large plasmid Fragment was separated from the smaller EcoRI-XbaI fragment by PAGE and isolated after electroelution. This DNA fragment from pHS32 (0.2 »g), was ligated, under conditions similar to those described above, to the EcoRI-Taq I fragment of the tryptophan operon ( 0.01 »g). In this process the Taq I protruding end is ligated to the XbaI remaining protruding end even though it is not completely Watson-Crick base-paired: 
——T              CTAGA——              ——TCTAGA——
          +                  →
——AGC              TCT——              ——AGCTCT——
A portion of this ligation reaction mixture was transformed into E. coli 294 cells as in part I. above, heat treated and plated on LB plates containing ampicillin. Twenty-four colonies were selected, grown in 3 ml LB media, and plasmid isolated. Six of these were found to have the XbaI site regenerated via E. coli catalyzed DNA repair and replication: 
——TCTAGA——          ——TCTAGA——
                  →
——AGCTCT——          ——AGATCT——
These plasmids were also found to cleave both with EcoRI and HpaI and to give the expected restriction fragments. One plasmid 14, designated pTrp 14, was used for expression of heterologous polypeptides, as next discussed.

The plasmid pHGH 107 (D.V. Goeddel et al, Nature, 281, 544, 1979) contains a gene for human growth hormone made up of 23 amino acid codons produced from synthetic DNA fragments and 163 amino acid codons obtained from complementary DNA produced via reverse transcription of human growth hormone messenger RNA. This gene, 3, though it lacks the codons of the "pre" sequence of human growth hormone, does contain an ATG translation initiation codon. The gene was isolated from 10 »g pHGH 107 after treatment with EcoRI followed by E. coli polymerase I Klenow fragment and dTTP and dATP as described above. Following phenol and chloroform extraction and ethanol precipitation the plasmid was treated with BamHI. The human growth hormone ("HGH") gene-containing fragment 3 was isolated by PAGE followed by electroelution. The resulting DNA fragment also contains the first 350 nucleotides of the tetracycline resistance structural gene, but lacks the tetracyline promoter-operator system so that, when subsequently cloned into an expression plasmid, plasmids containing the insert can be located by the restoration of tetracycline resistance. Because the EcoRI end of the fragment 3 has been filled in by the Klenow polymerase I procedure, the fragment has one blunt and one sticky end, ensuring proper orientation when later inserted into an expression plasmid.

The expression plasmid pTrp14 was next prepared to receive the HGH gene-containing fragment prepared above. Thus, pTrp14 was XbaI digested and the resulting sticky ends filled in with the Klenow polymerase I procedure employing dATP, dTTP, dGTP and dCTP. After phenol and chloroform extraction and ethanol precipitation the resulting DNA was treated with BamHI and the resulting large plasmid fragment isolated by PAGE and electroelution. The pTrp14-derived fragment had one blunt and one sticky end, permitting recombination in proper orientation with the HGH gene containing fragment 3 previously described. The HGH gene fragment 3 and the pTrp14 Xba-BamHI fragment were combined and ligated together under conditions similar to those described above. The filled in XbaI and EcoRI ends ligated together by blunt end ligation to recreate both the XbaI and the EcoRI site: 
XbaI filled in    EcoRI filled in    HGH gene initiation
___TCTAG    +    AATTCTATG___    ___TCTAGAATTCTATG___
___AGATC    TTAAGATAC___    →    ___AGATCTTAAGATAC___
XbaI    EcoRI
This construction also recreates the tetracycline resistance gene. Since the plasmid pHGH 107 expresses tetracycline resistance from a promoter lying upstream from the HGH gene (the lac promoter), this construction, designated pHGH 207, permits expression of the gene for tetracycline resistance under the control of the tryptophan promoter-operator. Thus the ligation mixture was transformed into E. coli 294 and colonies selected on LB plates containing 5 »g/ml tetracycline.

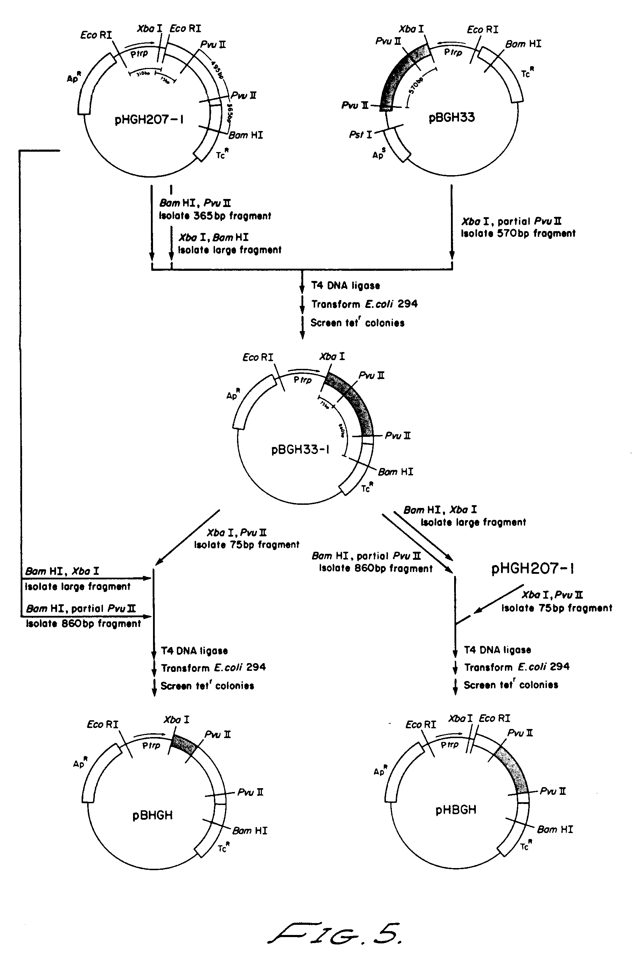

### Construction of pBGH33-1 (Figure 5)

The structure of pHGH207-1 which has the entire human growth hormone gene sequence is shown. The front part of this gene is synthetic as is described by Goeddel et al., Nature 281, 544 (1979). In the following a plasmid was constructed containing the BGH gene in the same orientation and in the same position with respect to the trp-promotor as is the HGH gene in pHGH 207-1.

Twenty »l (i.e. 10»g) of the plasmid DNA was digested wth Bam HI and PvuII as follows: To the twenty »l of DNA we added 5 »l 10X restriction enzyme buffer (500mM NaCl, 100 mM Tris HCl pH 7.4, 100 mM MgSO₄ and 10 mM DTT), 20 »l H₂O and 10 units BamHl restriction enzyme and 2 »l PvuII restriction enzyme. Subsequently, this reaction mixture was incubated at 37°C for 90 minutes. The mixture was loaded on a 6 % v/v acrylamide gel and electrophoresis was carried out for 2 hours at 50 mA. The DNA in the gel was stained with Ethidium bromide and visualized with UV-light. The band corresponding to the 365 bp (with reference to a HaeIII digest of pBR322) fragment was excised and inserted in a dialysis bag and the DNA was electroeluted using a current of 100 mA. The liquid was removed from the bag and its salt concentration adjusted to 0.3M NaCl. Two volumes of ethanol were added and the DNA precipitated at -70°C. The DNA was spun down in an Eppendorf centrifuge, washed with 70 % v/v ethanol and dried and resuspended in 10 »l TAE (10 mM Tris HCl pH7.4, 0.1 mM EDTA). Similarly, the large XbaI Bam Hl fragment of pHGH 207-1 and the XbaI, partial PvuII 570 bp fragment of pBGH33 were isolated.

Two »l of each of the thus isolated DNA fragments were mixed. 1 »l 10mM ATP and 1 »l 10x ligase buffer (200 mM Tris HCl pH7.5, 100mM MgCl₂, 20 mM DTT) and 1 »l T₄ DNA ligase and 2 »l H₂O were added. Ligation was done over night at 4°C. This mixture was used to transform competent E. coli K-12 294 cells as follows: 10 ml L-broth was inoculated with E. coli K-12 294 and incubated at 37°C in a shaker bath at 37°C. At OD₅₅₀ of 0.8 the cells were harvested by spinning in a Sorvall centrifuge for 5 min. at 6000 rpm. The cell pellet was washed/resuspended in 0.15 M NaCl, and again spun. The cells were resuspended in 75 mM CaCl₂, 5 mM MgCl₂ and 10 mM Tris HCl pH7.8 and incubated on ice for at least 20 min. The cells were spun down for 5 min at 2500 rpm and resuspended in the same buffer. To 250 »l of this cell suspension each of the ligation mixtures was added and incubated for 60 min on ice. The cells were heat shocked for 90 seconds at 42°C, chilled and 2 ml L-broth was added. The cells were allowed to recover by incubation at 37°C for 1 hour. 100 »l of this cell suspension was plated on appropriate plates which were subsequently incubated over night at 37°C. The plasmid structure in several of the colonies thus obtained is shown in Figure 5 (pBGH 33-1).

All further constructions were done using the same procedures, as described above, mutatis mutandis.

### Construction of the hybrid growth hormone genes HBGH and BHGH (Figure 5)

The two PvuII sites in the HGH and BGH genes are at identical positions. Exchange of PvuII fragments is possible without changing the reading frame of the messenger RNA of these genes. The large difference in expression of both genes is due to differences in initiation of protein synthesis at the beginning of the messages. Therefore, the front part of both genes were exchanged thus constructing hybrid genes that upon transcription would give hybrid messenger RNAs. The two plasmids, pBHGH and pHBGH, were constructed as follows:

From pHGH207-1 there were isolated the large BamHI-XbaI fragment and the 857 bp BamHI (partial) PvuII fragment containing the HGH gene without its front part. From pBGH33-1 there was isolated the 75 bp XbaI-PvuII fragment that contains the front part of the BGH gene. After ligation and transformation pBHGH was obtained. pHBGH was constructed in a similar way to pBHGH; in this case the back part was derived from pBGH33-1 whereas the front part, the 75 bp XbaI-PvuII fragment, was derived from pHGH207-1.

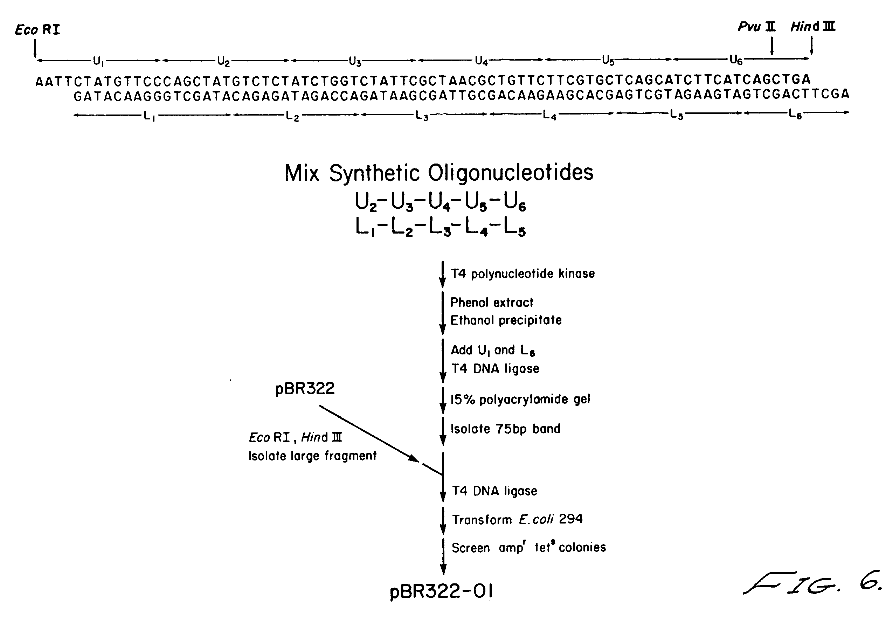

### Design and cloning of the synthetic front part of the BGH gene (Figure 6)

The DNA sequence up to the PvuII site of the BGH and HGH genes codes for 22 amino acids. Since the front part of the HGH gene had excellent protein synthesis initiation properties, the sequence of the front part of BGH was designed such that the number of nucleotide changes in the BGH gene would be minimal with respect to the HGH gene. Only 14 base pair changes from the natural BGH sequence were made in order to code for the proper BGH amino acid sequence and reduce conformational structure in the prospective mRNA. The DNA sequence is shown in Figure 6. The sequence ends with EcoRI and HindIII sticky ends to make cloning in a vector easy. Close to the HindIII site is a PvuII site for the proper junction with the remaining part of the BGH gene.

The fragments U1 to U6 and L1 to L6 were synthesized chemically according to the procedures described above. All the fragments except U1 and L6 were mixed and kinased. After addition of U1 and L6 the mixed fragments were ligated, purified on a 6 % v/v polyacrylamide gel and the 75 bp band extracted and isolated according to standard procedures. This fragment was inserted into pBR322 that had been cut with EcoRI and HindIII. Thus plasmid pBR322-01 was obtained.

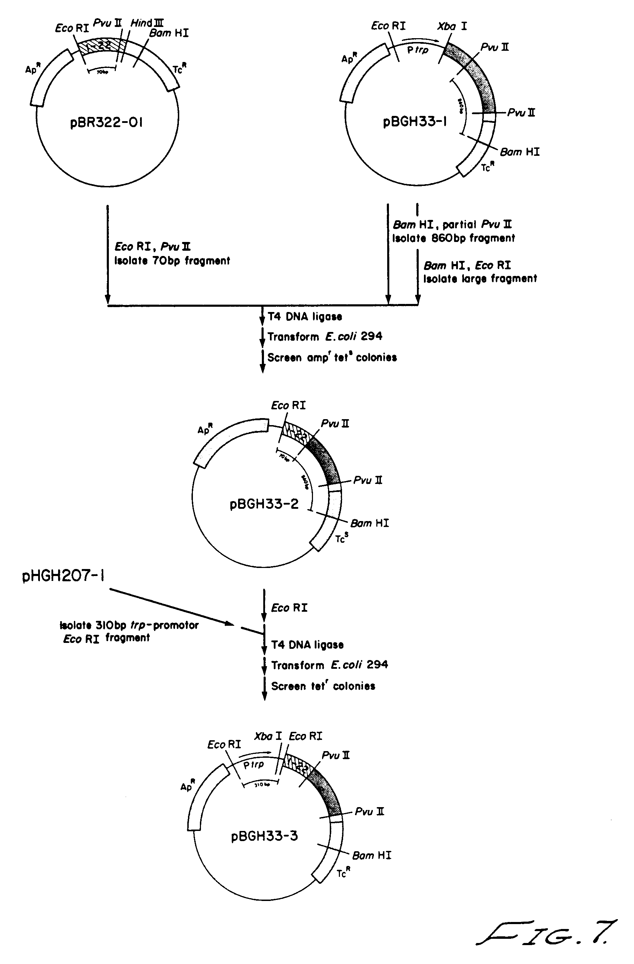

### Replacement of the natural front part of the BGH gene by the synthetic front part. (Figure 7)

From pBR322-01 the cloned synthetic front of the BGH gene was excised with EcoRI and PvuII, and the resulting 70 bp fragment was isolated. From pBGH33-1 the large EcoRI-BamHI fragment and the 875 bp BamHI (partial) PvuII fragment was isolated. The three fragments were isolated and ligated and used to transform E. coli K-12 294 as described before. Thus, pBGH33-2 was obtained. This plasmid contains the entire BGH gene but does not have a promotor. Therefore, pBGH33-2 was cut with EcoRI and the trp-promotor containing 310 bp EcoRI fragment derived from pHGH207-1 was inserted by ligation. After transformation tetracycline resistant colonies were analyzed. Therefore, these colonies had the inserted trp-promotor oriented towards the HGH- and tet-gene as shown in the figure.

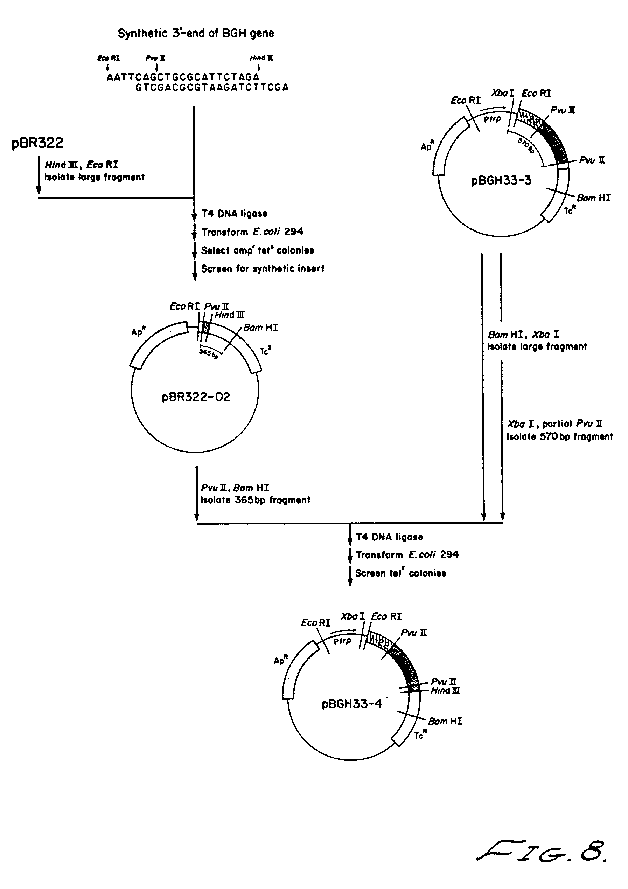

### Repair of the 3ʹ-end of the BGH gene. (Figure 8)

The sequences beyond the second PvuII site of the BGH gene are derived from the HGH gene. One of the amino acids at the end is different from that in the natural BGH gene. This 3ʹ-end was repaired as follows. A synthetic DNA fragment as shown was synthesized. It is flanked by an EcoRI and a HindIII end to facilitate cloning and contains a PvuII site and 3 amino acid codons and a stop codon in the reading frame of the BGH gene itself. This fragment was inserted into EcoRI-HindIII opened pBR322. Thus pBR322-02 was obtained. Subsequently this plasmid was cut with PvuII and BamHI and the 360 bp fragment was isolated. From pBGH33-3, which has the entire BGH gene with the synthetic front part, the large BamHI and XbaI fragment and the 570 bp XbaI (partial) PvuII fragment was isolated. These three fragments were ligated and used to transform cells. Thus, pBGH33-4 was obtained. In this plasmid a unique HindIII site is present between the stop codon of the BGH gene and the start codon of the tet-mRNA. Both genes are transcribed under direction of the trp promotor.

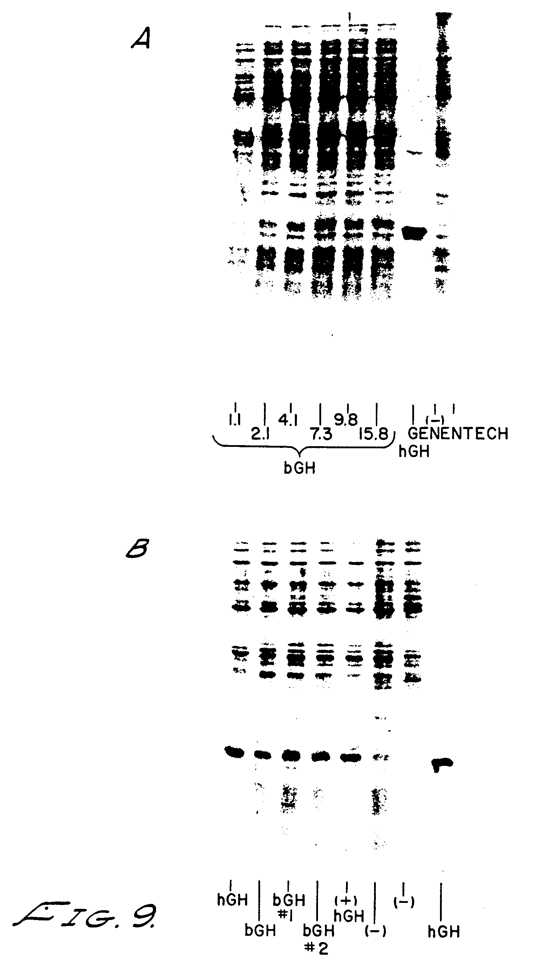

A typical growth medium used to derepress and produce high levels of BGH per liter (Figure 9) contains: 5.0 g (NH₄)₂SO₄, 6.0 g K₂HPO₄, 3.0 g NaH₂PO₄.2H₂O, 1.0 g sodium citrate, 2.5 g glucose, 5 mg tetracycline, 70 mg thiamine HCl, and 60 g MgSO₄.7H₂O.

While the present invention has been described, in its preferred embodiments, with reference to the use of E. coli transformants, it will be appreciated that other microorganisms can be employed mutatis mutandis. Examples of such are other E. coli organisms, e.g. E. coli B., E. coli W3110 ATCC No. 31622 (F⁻, λ-, gal⁻, prototroph), E. coli x 1776, ATCC No. 31537, E. coli D1210, E. coli RV308, ATCC No. 31608, etc., Bacillus subtilis strains, Pseudomonas strains, etc. and various yeasts, e.g., Saccharomyces cerevisiae many of which are deposited and (potentially) available from recognized depository institutions e.g., ATCC. Following the practice of this invention and the final expression of intended polypeptide product, extraction and purification techniques may be those customarily employed in this art, known per se.

## Claims

1. A microorganism transformed with a BGH gene having a synthetic 5' terminal region to the BGH coding sequence, the DNA sequence of said synthetic region differing from the native coding sequence by having less potential for the formation of mRNA secondary structure, so that the microorganism is capable of producing BGH in amounts exceeding 100,000 copies per cell.

2. A process which comprises culturing a microorganism according to claim 1, and producing therein BGH in amounts exceeding 100,000 copies per cell.

## Patentansprüche

1. Mikroorganismus, der mit einem BGH-Gen transformiert ist, das einen synthetischen 5'-terminalen Bereich an der BGH-Kodierungssequenz aufweist, wobei sich die DNA-Sequenz des synthetischen Bereichs von der nativen Kodierungssequenz dadurch unterscheidet, daß sie weniger Potential zur Bildung von sekundärer mRNA-Struktur aufweist, sodaß der Mikroorganismus in der Lage ist, BGH in Mengen zu erzeugen, die 100 000 Kopien pro Zelle übersteigen.

2. Verfahren, welches das Kultivieren eines Mikroorganismus nach Anspruch 1 und das Herstellen von BGH darin in Mengen über 100 000 Kopien pro Zelle umfaßt.

## Revendications

1. Micro-organisme transformé par un gène BGH dont la séquence codant pour le BGH comporte à l'extrémité 5' une région synthétique, la séquence d'ADN de ladite région synthétique étant différente de la séquence codante native en ce qu'elle diminue la tendance de l'ARNm à former une structure secondaire, de sorte que le micro-organisme est capable de produire du BGH en quantités supérieures à 100 000 copies par cellule.

2. Procédé qui comprend la mise en culture d'un micro-organisme selon la revendication 1, en vue de produire du BGH en quantités supérieures à 100 000 copies par cellule.
